Europäisches Patentamt

European Patent Office

Office européen des brevets

Publication number: **0 299 359**

**A2**

# EUROPEAN PATENT APPLICATION

Application number: **88110859.1**

Int. Cl.⁴ **G01N 33/543**

Date of filing: **07.07.88**

Priority: **16.07.87 US 73932**

Date of publication of application:
**18.01.89 Bulletin 89/03**

Designated Contracting States:
**AT BE CH DE ES FR GB GR IT LI LU NL SE**

Applicant: **ABBOTT LABORATORIES**

**Abbott Park Illinois 60064(US)**

Inventor: **Korom, Gordon Keith**
**790 Durham Lane**
**Grayslake Illinois 60030(US)**
Inventor: **Caplan, Diane Elizabeth**
**605 Smith Avenue**
**Lake Bluff Illinois 60044(US)**
Inventor: **Eng, Katherine Kit-Ming**
**220 Kenloch Avenue**
**Libertyville Illinois 60048(US)**
Inventor: **Hansa, Janet Gail**
**1 Garden Drive, No. 6**
**LaGrange Park Illinois 60525(US)**

Representative: **Modiano, Guido et al**
**MODIANO, JOSIF, PISANTY & STAUB**
**Modiano & Associati Baaderstrasse 3**
**D-8000 München 5(DE)**

Reagent delivery system for use in solid-phase analytical devices.

A reagent delivery system for use in solid phase analytical devices is disclosed. Matrices containing a reagent dispersed in a water soluble polymer that controls the dissolution rate are positioned over a reaction matrix such that sample liquid dissolves the conjugate to perform a solid-phase immunoassay reaction. The matrices are then removed from the reaction matrix where a detectable signal is formed for the particular solid-phase immunoassay performed.

*FIG. 1*

# REAGENT DELIVERY SYSTEM FOR USE IN SOLID-PHASE ANALYTICAL DEVICES

## BACKGROUND OF THE INVENTION

The present invention relates to analytical devices and methods for their use. More particularly. the present invention relates to a reagent delivery system for diagnostic devices whereby reagents suitable for performing an assay are dispersed in a plurality of matrices. The subject invention allows for the elimination of reagent addition steps normally associated with the performance of a solid-phase immunoassay. Concepts of the present invention are especially advantageous in the performance of enzyme immunoassay of biological fluids such as serum, plasma, whole blood, urine, spinal and amniotic fluids, mucus, and the like.

Recently. the trend has been to develop diagnostic devices which are simple to use and are highly accurate. The emphasis has been to provide diagnostic devices which can be used by both medical and non-medical personnel with minimal training. Devices which have attempted to provide safe, efficient and reliable diagnostic tests are disclosed in numerous U.S. Patents 3.888.629. 4.246.339. 4.407.943 4.515.889. 4.425.438. and 4.366.341.

For example, U.S. Patent 4.632.901 describes an apparatus having a membrane to which is bound a receptor for an analyte. Sample is added to the membrane followed by the manual addition of reagents and washes for identifying the analyte. Typically. in such a diagnostic ʼˑˑʼice. a sample is first added followed by a labeʾˑ antibody, a buffered wash and a substance capable of forming a detectable signal in the presence of a labeled antibody. It is not uncommon. however, that additional reagents must be added prior to the determination of the analyte. In addition, there are usually requirements that each reagent be added at a predetermined time.

Other problems associated with such diagnostic devices is that each reagent must be separately packaged and identified. Also accidental use of the wrong reagent or even the right reagent at the wrong time can ruin the assay. It is therefore desirable to eliminate steps which can be subject to error.

One attempt to solve this problem is described in International Patent Application No. PCT U.S. 86 01603 which discloses a diagnostic device having a shaped porous filter which may have reagent dispersed therein positioned over a porous member having a bound analyte receptor. Incorporation of the reagent into the shaped porous mass is achieved by addition of a quantity of solution less than that which will completely saturate the shaped mass followed by a drying operation. preferably lyophilization. Undesirable features of this method is that control of the reagent's addition to the reaction matrix is very limited and lyophilization is a costly manufacturing method. It is therefore desirable to develop improved methods for applying a reagent system to a reaction matrix whereby a diagnostic assay can be completely performed using minimal steps.

## Summary of the Invention

The present invention is directed toward a reagent delivery system suitable for use in a diagnostic device such as an solid-phase immunoassay apparatus. The reagent delivery system is a matrix saturated with a reagent containing water soluble polymer that controls the dissolution rate of the reagent for delivery to a reaction matrix where an assay is performed. In a diagnostic device the matrix is in intimate contact with the reaction matrix.

Typically the reagent is an antibody, chromogen, pretreatment agent, buffer or any combination thereof. Preferably the water soluble polymer is a polyvinyl pyrrolidone.

In another aspect. the present invention is a diagnostic device for performing an assay comprising a plurality of matrices containing various components of a reagent system dispersed in a water soluble polymer that controls the dissolution rate of the reagent component for delivery to a reaction matrix where the assay is performed. The matrices are in intimate contact with one another and the reaction matrix. The matrices of the diagnostic device can be saturated with various components of a reagent system such as is an antibody. chromogen. pretreatment agent. buffer or any combination thereof.

The present invention provides the advantage of reducing the number of reagent addition steps that must be performed for an assay. Also. stability and packaging problems associated with the reagent are reduced because the reagent is dried in the various matrices.

## Brief Description of the Drawings

Figure 1 is a cross-sectional view of a solid-phase diagnostic device depicting one embodiment of the reagent delivery system.

Figure 2 is prospective view of a solid-phase diagnostic device for use with the reagent delivery system.

Figure 3 is a cross-sectional view of the diagnostic device of Fig. 2 with a reagent delivery system.

Detailed Description of the Invention

The present invention provides a reagent delivery system having reagent dispersed in a soluble polymer phase contained in a matrix positioned over and in intimate contact with a reaction matrix for performing an assay. The reagent delivery system is suitable for use in solid-phase assays generally including, but not limited to immunoassays which use antigen, polyclonal or monoclonal antibody as a reagent and DNA probe assays which use nucleic acid oligomer as a reagent. The invention is particularly suitable for use in immunometric assays which employ a labeled antibody or antigen as a component of the reagent system.

The articles of the subject invention generally comprise a matrix saturated with a reagent or components thereof dispersed in a water soluble polymer for controlling the dissolution rate of the reagent for delivery to a reaction matrix positioned below the matrix. The water soluble polymer containing a reagent and dispersed throughout a matrix acts as a time release mechanism for adding the reagent to the sample and ultimately to the reaction matrix. This reagent delivery system was developed to eliminate reagent addition steps normally associated with the performance of solid-phase immunoassay.

For example, the first step in a typical solid-phase immunoassay involves the addition of a sample to a reaction matrix having an immobilized antibody which binds analyte present in the sample. The next step involves adding a conjugate which can consist of an enzyme-labeled antibody to the reaction matrix and incubating for approximately one minute. Excess conjugate is then washed away and a chromogen is added and incubated to allow quantitation of the bound analyte. After another incubation period, excess chromogen is washed away to reveal a detectable signal on the reaction matrix.

In one aspect of the present invention, the second step of a typical solid-phase acid protocol can be eliminated, i.e., the addition of a liquid conjugate. This is accomplished by dissolving the conjugate in an aqueous solution containing a water soluble polymer, saturating a matrix with this solution, and drying the solution on the matrix. The reagent containing matrix is then placed in contact with the upper surface of a reaction matrix or in contact with a membrane which is in contact with the reaction matrix. "Reaction matrix" is defined as the solid support of a diagnostic device where the analyte is captured and detected i.e., where the assay is ultimately performed. When sample is added to the diagnostic device, the liquid dissolves the conjugate and allows the assay reaction to begin.

A critical feature of the present invention is that the reagent must be released at a predetermined rate such that it is not washed through the reaction matrix before the assay reaction with that particular reagent can occur, i.e. an adequate incubation period. The assay reaction includes the capture of the analyte and the associated reaction with the released reagent. The incubation period for the reaction to occur is generally determined by the dissolution rate of the water soluble polymer.

Optionally, the incubation time can be increased by placing a membrane between the reagent containing matrix and the reaction matrix to increase the distance the reagent and sample must travel before contacting the reaction matrix. The mesh size of the membrane can also be selected to control the rate of fluid passage. Typically the mesh size of the membrane can vary from about 1 to about 30 microns in size, preferably the mesh size is smaller than the mesh size of the reagent containing matrix.

Alternate embodiments of present invention can include a plurality of matrices prepared to each contain a different reagent component of an assay system. For example, one of the matrices can contain the conjugate material while another can contain the chromogen, each being predissolved in a water-soluble polymer and applied to the matrix and dried. The dissolution rate of each would be predetermined to appropriately release the reagent such that the assay reaction can occur to effectively determine the presence of an analyte in a sample material being tested.

Two main features of the subject reagent delivery system are the composition of the reagent and water soluble polymer and the matrix which contains this composition. The matrix regulates the flow of the sample and the reagent onto the reaction matrix and provides a means to which the reagent composition can be adhered. The matrix for depositing the solution of reagent and water soluble polymer is chosen such that the pore site is large enough to allow liquid flow through the filter and to allow for the dissolution of the water soluble polymer. Generally the mesh size or pore size is from about 1 to about 30 microns in average diameter. The matrix can comprise fibrous materials or open cell materials. Examples of open cell materials can include molded or fused plastics, polymers or glass. The matrices can be composed bibulous or non-bibulous fibers, and synthetic or natural fibers such as glass or cellulose. Commercially available matrices are Lydall #254 (Lydall Inc.. Manchester, CN) and Whatman GF F and GF D (Whatman Reeve Angel Inc.. Clifton. NJ).

The composition of the reagent and water soluble polymer is prepared such that the reagent is solubilized at a predetermined rate calculated by the dissolution rate of the water-soluble polymer. The dissolution rate of a water-soluble polymer is dependent on the molecular weight of the polymer. This information is generally available from the manufacturer. For example. polyvinyl pyrrolidone (PVP) having a molecular weight of 30.000 can be employed as a 10% solution with a reagent to yield a 30 second to one minute dissolution rate. while a 300.000 MW of PVP in a 1% solution will yield the same dissolution rate. Typically a polymer is selected to provide a dissolution rate of about 30 seconds to about 2 minutes.

For purposes of this invention. reagent is used to mean a variety of components that are normally associated in the performance of a solid-phase immunoassay. For example, a reagent system generally includes a conjugate which is an enzyme-labeled antibody or an antibody, a chromogen. pretreatment agents or buffers that are necessary to develop a detectable assay signal for an analyte.

An additional benefit of the reagent delivery system is that it can be used to indicate when the immunoassay reaction has been completed by observing a change in color of the matrix saturated with a time-release reagent. After a color change has occurred the matrix can be removed to reveal the assay result in the reaction matrix. A color change obtained by the natural reaction of the sample fluid or other reagent with the reagent contained in the saturated matix. Alternatively, a dye or other chromogenic material can be incorporated into the time-release reagent to artificially generate a color change.

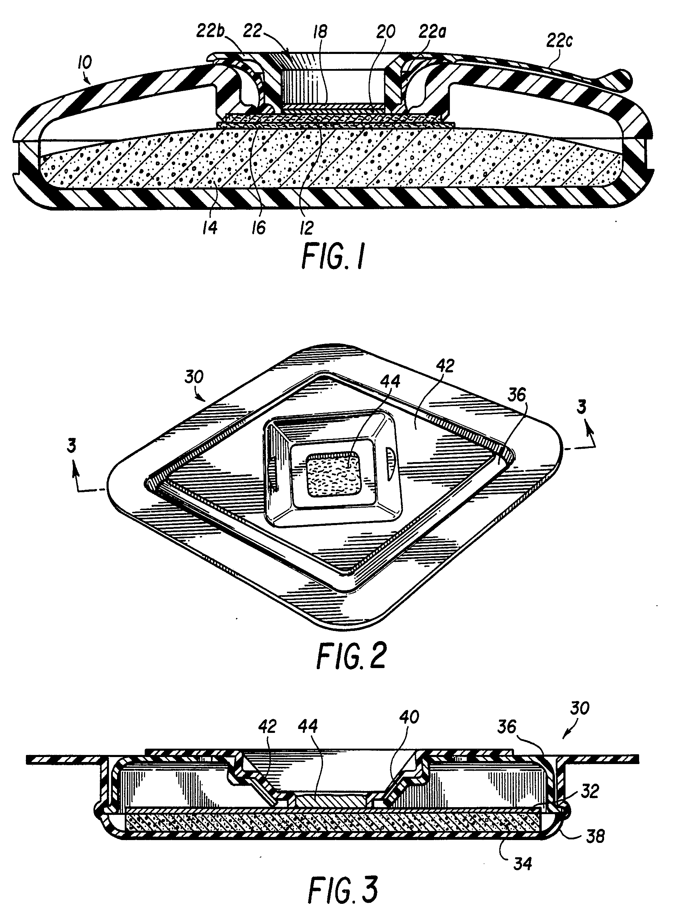

In one preferred embodiment. the reagent delivery system can be adapted for use with a solid-phase analytical device as disclosed in EP 0217403. herein incorporated by reference. This device is commercially available as TESTPACK® from Abbott Laboratories. North Chicago. Illinois. and is only shown here for purposes of describing one use for the subject reagent delivery system. A cross-section view of this device is shown in Fig. 1. The device 10 includes a reaction matrix 12 positioned over an absorbent member 14 for absorbing fluid and assisting sample and reagent fluids to flow through the reaction matrix 12. The device 10 further depicts a barrier material 16 in contact with the lower surface of the reaction matrix 12 and the upper surface of the absorbent member 14. The barrier material 16 functions to restrict fluid from passing from the absorbent member 14 back into the reaction matrix 12. The matrix saturated with a time-release reagent composition 18 is positioned directly over and in contact with a membrane 20 which is in direct contact with the reaction matrix 12. The matrix saturated with a time-release reagent composition 18 and the membrane 20 are press-fitted into the carrier 22 disposed over the surface of the reaction matrix 12. The carrier 22 is press-fitted into the device 10 by means of a retaining ring 22a. and is removable by a handle or other means 22c for removing both the matrix 20 and membrane 18 such that a detectable signal can be read off the surface of the reaction matrix 12.

Another embodiment of the subject reagent delivery system is shown in Figures 2 and 3. The solid-phase diagnostic device 30 comprises a reaction matrix 32 positioned over an absorbent member 34 for absorbing fluid and assisting sample and reagent fluids to flow through the reaction matrix 32. The device 30 has a molded top surface 36 which engageingly fits into a molded lower surface 38. The top surface 36 further provides a well means 40 which provides access to the reaction matrix 32 and an engaging means for a molded filter housing 42 to be placed. The filter housing 42 is designed to receive a plurality of matrices containing the time-release reagent compositon and or membranes. The device depicted in Figures 2 and 3 shows the filter housing 42 with a matrix containing the time-release reagent composition 44. When the filter housing is positioned in the well 40 the filter matrix 44 will be in direct contact with the reaction matrix 32.

In one embodiment, for example in the performance of a sandwich assay. the user would obtain a device with the plastic filter housing inserted into the well and pour sample into the housing. After approximately 1 minute the sample will have flowed through the matrix dissolving the reagent from the fibrous matrix for reaction with a bound receptor in the reaction matrix. The filter housing is then removed and a wash and an appropriate chromogen is added to form a signal on the reaction matrix.

The matrices containing a time-release reagent are prepared by first dissolving the particular reagent in an aqueous solution containing a water soluble polymer. Water soluble polymers can comprise polyvinyl pyrrolidone, polyvinyl alcohol. gum arabic. and gum tragacanth. After the reagent is dispersed in a water soluble polymer. the fibrous matrix is dipped into the solution to completely saturate the fibers. The fibrous matrix is them removed from the solution and dried. Drying can be conducted by any conventional means such as by forced air or by the application of heat either by forced air or in an oven. Even though the reagent material can be lyophilized drying is the preferred method.

The saturated matrix is placed in contact with the reaction matrix surface or in contact with a membrane which is in contact with the reaction matrix of an analytical device such that when the sample or other liquid reagent is added, the liquid will dissolve the reagent and allow the solid phase immunoassay reaction to begin.

The reagent and water soluble polymer solution is prepared such that when it is dried into the matrix, the dissolution rate of the reagent will be sufficient to allow adequate time for the sample to react with the contained reagent and the bound receptor present on the reaction matrix. In other words, the reagent must be released from the matrix at a rate such that an adequate incubation period is provided whereby the sample and reagent and bound receptor can react. Generally, this time interval is approximately thirty seconds to about two minutes for most solid-phase immunoassays. An appropriate solution for dispersing the reagent has been determined to be from about five to about twenty percent by weight of an aqueous solution of a water soluble polymer depending on the molecular weight as discussed above.

After the matrix is saturated with the reagent and water-soluble polymer and dried, it can be positioned on a reaction matrix of a solid phase analytical device or in otherwise capillary communication with the reaction matrix. It is important that the matrix be in intimate contact with the reaction matrix. "Intimate contact" means that the matrices of the present invention are in at least capillary communication with the reaction matrix either directly or by additional membranes inserted between the reaction matrix and the matrices containing the time-release reagent composition. This is important to provide a uniform flow of the sample and thereby dissolved reagent into the reaction matrix. It also avoids droplets forming at the interface of the matrix and the reaction matrix which can cause a non-uniform flow of the fluid through the reaction matrix. In instances where multiple matrices are employed, each having different reagents deposited therein, it is important that each of the matrices be placed in contact one with the other to provide a uniform flow to the reaction matrix.

It is envisioned that multiple matrices, each having a separate reagent incorporated therein, can be employed with an analytical device. In fact, all the reagents can be contained in the analytical device in the matrices such that the entire assay can be performed by merely adding a sample to the diagnostic device. The arrangement of the matrices over the reaction matrix is not critical and can therefore encompass a wide degree of variation depending on the intended use. In fact, matrices having reagents dispersed therein can be separated one from the other by other membranes not having any reagent dispersed therein for purposes of providing additional incubation time or as a filter mechanism to filter out large particles which may be present in the sample material.

To further illustrate the subject invention, the following examples are provided to describe methods for preparing the matrix and use of the matrix in a solid phase analytical device.

EXAMPLE 1

Preparation of a Matrix Containing an Antibody-Conjugate

An aqueous solution was prepared in a one-liter flask containing 10% polyvinyl pyrrolidone (30.000 MW), 1% lactose, 1% bovine serum albumin, 0.1% of a surfactant (Brij 35 a surfactant commercially available from Aldrich Chemical Co., Milwaukee, Wisconsin), 0.1% sodium azide, 15 mM of tris (hydroxymethyl)-aminomethane, 1 mM magnesium chloride, and 0.1 nM zinc chloride. All percentages are by weight per volume and mM is millimolar. The aqueous solution was adjusted to a pH of 7.5 and 100 micrograms per milliliter of anti-hCG antibody labeled with a alkaline phosphatase (0.1 grams) was added.

A strip of Lydall #254 (commercially available from Lydall Inc., Manchester, CT) having a 10 micron mesh was saturated with the above prepared solution and dried in an oven at 45° C after drying the strip was cut into a disk shape for placement in a solid-phase assay device.

EXAMPLE 2

Solid-Phase Immunoassay Employing a Conjugate Saturated Matrix

The saturated and dried fibrous matrix of Example 1 was positioned above and in contact with a reaction matrix containing bound hCG antibody. Approximately 400 microliters of urine was added to the saturated fibrous matrix and reaction matrix assembly similar to the solid-phase device shown in Fig. 2 and 3. After a 2 minute incubation step the saturated fibrous matrix was removed and a buffered wash was applied to the reaction matrix. A detectable signal was formed on the reaction matrix by adding 150 microliters of chromogen and incubating for an additional 2 minutes. A final wash was applied to remove excess chromogen.

## Claims

1. A diagnostic device for performing an assay characterized in that a matrix contains a reagent dispersed in a water soluble polymer that controls the dissolution rate of the reagent for delivery to a reaction matrix where the assay is performed.

2. The diagnostic device of Claim 1 wherein said matrix is in intimate contact with said reaction matrix.

3. The diagnostic device of Claim 1 wherein said water soluble polymer is polyvinyl pyrrolidone.

4. A diagnostic device for performing an assay characterized in that a plurality of matrices contain a plurality of reagents dispersed in a water soluble polymer that controls the dissolution rate of the reagents for delivery to a reaction matrix where the assay is performed.

5. The diagnostic device of Claim 4 wherein said matrices are in intimate contact with one another and said reaction matrix.

6. The diagnostic device of Claims 1 or 4 wherein the reagent comprises antibody, chromogen, pretreatment agent, buffer or any combination thereof.

7. The diagnostic device of Claim 4 wherein said water soluble polymer is polyvinyl pyrrolidone, polyvinyl alcohol, gum arabic or gum tragacanth.

8. The diagnostic device of Claim 4 wherein a first matrix contains an antibody for performing an assay and a second fibrous matrix containing a chromogen and buffer.

9. The diagnostic device of Claim 4 wherein a first matrix contains a pretreatment agent for a sample, a second fibrous matrix contains an antibody and a third fibrous matrix contains a chromogen.

10. The diagnostic device of Claim 4 which includes at least one membrane positioned between two matrices or positioned between a matrix and said reaction matrix.

FIG. 1

FIG. 2

FIG. 3